# EUROPEAN PATENT SPECIFICATION

(11) **EP 3 930 713 B1**
(45) Date of publication and mention of the grant of the patent: **28.05.2025**
(21) Application number: 18942402.1
(22) Date of filing: 04.12.2018
(51) Int. Cl.: A61K 9/06, A61K 47/06, A61K 47/14, A61K 47/44, A61K 47/12, A61K 31/436

(54) **STABLE TACROLIMUS OINTMENT FORMULATION FOR TOPICAL TREATMENT OF SKIN CONDITIONS**
STABILE TACROLIMUS-SALBENFORMULIERUNG ZUR TOPISCHEN BEHANDLUNG VON HAUTERKRANKUNGEN
FORMULATION DE POMMADE À BASE DE TACROLIMUS STABLE POUR LE TRAITEMENT TOPIQUE D'AFFECTIONS CUTANÉES

(43) Date of publication of application: 05.01.2022
(73) Proprietor: Ilko Ilaç Sanayi Ve Ticaret Anonim Sirketi, Sancaktepe/Istanbul (TR)
(72) Inventor: ÖNCEL, Hatice, Sancaktepe/Istanbul (TR); ÇAPAN, Yilmaz, Çankaya/Ankara (TR); PINARBASLI, Onur, Cankaya/Ankara (TR); SARRAÇOGLU, Nagehan, Cankaya/Ankara (TR)
(86) International application number: PCT/TR2018/050759
(87) International publication number: WO 2020/117134

(56) References cited:
- EP-A1- 0 474 126
- EP-A1- 2 345 414
- CN-A- 106 166 138

## Description

### Technical field:

The present invention relates to formulate a stabilized pharmaceutical dermal composition comprising tacrolimus. In particular, the present invention relates to storage stable Tacrolimus ointment composition comprises tacrolimus and specific weight percentage of propylene carbonate based on the total composition rather than using a stabilizing agent; which shows acceptable degradation product percent upon storage.

### Prior Art:

Tacrolimus is a member of macrolides immunosuppressant with chemical name (3S-{3R',(E(1S',3S',4S')),4S',5R',8S',9E,12R',14R',15S',16R',18S',19S',26aR'))-5,6,8,11, 12,13,14,15,16,17,18,19,24,25,26,26a-hexadecahydro-5,19-dihydroxy-3-(2-(4-hydroxy-3- methoxycyclohexyl)-1-methylethenyl)-14,16-dimethoxy-4,10,12,18-tetramethyl-8-(2-propenyl)-15,19-epoxy-3H-pyrido(2,1-c)[1,4]oxaaza-cyclotricosine-1,7,20,21(4H,23H)-tetrone monohydrate. It has been isolated from culture of Streptomyces tsukubaensis. Its molecular formula is C₄₄H₆₉NO₁₂ and it is represented by Formula I (shown below); 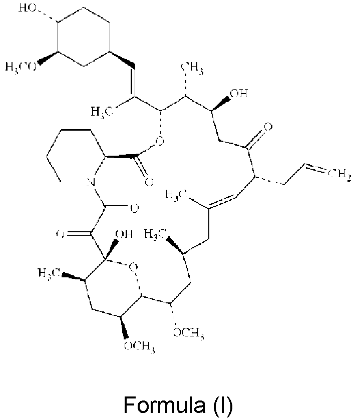 Tacrolimus appears in the form of white crystals or crystalline powder. It is a poorly soluble substance; whereas practically insoluble in water, freely soluble in ethanol and very soluble in methanol and chloroform.

The preparation and immunosuppressant and antimicrobial activity of tacrolimus are described in EP0184162B1 (Fujisawa). It is useful for the treatment and prevention of various autoimmune diseases such as organ or tissue transplant rejection or graft vs host disease, infection diseases and the like.

EP2575769B1 relates to an oral dosage form of tacrolimus with using tartaric acid as a stabilizing agent useful for preventing or reducing the formation of tacrolimus degradation products upon storage.

WO2005/020993 discloses tacrolimus formulations which may be useful in combination with the stabilizing agent(s) disclosed herein to yield a stabilized tacrolimus composition.

Tacrolimus is also useful for the treatment of cutaneous diseases such as atopic dermatitis. Atopic dermatitis (AD), also known as eczema, is an inflammatory skin disease typified by itchy inflamed skin that presents during infancy and childhood. The disease is one of the most common dermatological disorders in Western countries, affecting as many as 20% of children under the age of five. Cookson WO et al., Nature Genetics 27: 372-373 (2001).

Recently, there has been a significant increase in the number of patients suffering from atopic dermatitis. It has been known that skin of a patient suffering from atopic dermatitis has lower contents of epidermal lipid and keratoid moisture, lesser ability to form hydrolipidic film, and lower resistance threshold against external irritations comparing to those of normal subjects. Furthermore, it has also been known that abnormal-dryness or pruritus of skin results from a wreck of barrier function of skin. Therefore, there is a need for an external preparation comprising tacrolimus.

While tacrolimus presents clear advantages over corticosteroids in terms of its tolerance profile, formulating tacrolimus for topical application has proved challenging. First difficulty that arises is obtaining a chemically and physically stable product. Second one that arises is managing to deliver a sufficient amount of the active through the outer skin barrier for it to be efficacious. Permeation of tacrolimus is hindered by its molecular size (804 Da), which is far greater than the commonly recognised readily permeable size of 500 Da.

There has been tacrolimus containing external preparations, for example ointment, lotion, cream and gel. However there are technical problems that the active ingredient tacrolimus is decomposed during long-term storage. The presence of degradation products in a pharmaceutical formulation, including a pharmaceutical composition comprising tacrolimus as an active pharmaceutical ingredient, is highly undesirable, since it imposes an increased risk to the patients.

EP0474126B1 represents an ointment comprising a tricyclic compound, tacrolimus and a specific solubilizing and/or absorption-promoting agent. Tacrolimus or its pharmaceutically acceptable salt is used in an amount of about 0.01 to 10% (w/w), the solubilizing and/or absorption-promoting agent, propylene carbonate, is used in an amount of about 1 to 30 % (w/w) and the remaining is balanced by ointment bases so as to become 100% in the ointment. In this invention, propylene carbonate weight percent has wide range in the formulation. In one example given in this patent, prepared formulation contains 0.1% tacrolimus and 5% of propylene carbonate. The product prepared in Example 19 was filled in phenol coat tube and kept at 40°C for a predetermined period of time. In stability test it was analyzed only for residual rate (%) of FK506. According to results given, residual rate (%) of FK506 was 94.0% after 3 months at 40°C. They are silent about the degradation of tacrolimus at high temperature. However there are known technical problems that the active ingredient tacrolimus is decomposed during long-term storage.

CN106166138 A discloses a tacrolimus paste preparation containing: (a) 0.03% w/w or 0.1% w/w tacrolimus, (b) 0.5-8.0% w/w propylene carbonate, (c) 0.5-4.0% paraffin, (d) 15-55% w/w liquid paraffin, (e) 1-6% w/w beeswax, (f) 29.9-59.9% w/w white Vaseline, (g) 0.005-0.01% w/w vitamin E. The ratio tacrolimus to propylene carbonate is preferably 1 to 5-80. The range of 0.5-8.0% propylene carbonate is much more broad with respect to range disclosed in the present invention. There are no short-term or long-term stability test results.

EP2345414B1 provides an external preparation in form of an ointment comprising tacrolimus as an active ingredient and triacetin as a solubilizer thereof. This invention compared test product and reference product (Protopic Ointment) by means of their stability. (Any other unspecified degradation product: 2.58% for Test product and 2.88% for Reference product after 1 week storage at 30°C)

EP2596788 discloses an oil-in-water type creamy composition comprising tacrolimus in which diisopropyl sebacate may be used in the oil phase. The long-term stability of the composition at elevated temperatures (e.g. 40°C) is not disclosed. Moreover, in order to achieve sufficient permeation of the active, it is typically necessary to include a high amount of surfactant and/or polar water-miscible liquid. This is exemplified by US2005/0249757, US2012/018451, and EP2596788.

For tacrolimus-containing formulations, there is a need for preventing the formation of degradation products from tacrolimus or, at least, to maintain an acceptable, low concentration of such degradation products throughout the shelf-life of the formulation, which typically is a formulation in a unit dosage form such as a capsule (soft or hard), a tablet, or granules in a sachet, or as an injection liquid, or as a topical product.

Therefore, the present invention provides a way of improving the stability of topical formulation of tacrolimus by identifying the critical ratios between active ingredient and excipients.

### Description of the Invention:

The present invention is directed to a stabilized pharmaceutical topical composition comprising therapeutically effective amount of tacrolimus. According to one embodiment of the invention, the ointment composition comprises therapeutically effective amount of tacrolimus or its pharmaceutically acceptable salts, esters, and solvates thereof as an active ingredient and propylene carbonate as a solvent with a specific weight percent based on the total composition.

According to main embodiment of the invention, storage stable topical ointment compositions comprise 0.1% or 0.03% (w/w) of tacrolimus or its pharmaceutically acceptable salts, esters, and solvates thereof as active ingredient and 6.0% to 7.0% (w/w) of propylene carbonate as a solvent based on the total composition rather than using a stabilizing agent.

As used herein, the term "topical composition" (synonymously, "topical formulation") refers to a pharmaceutical preparation intended for topical or local application to an afflicted region of a subject in need thereof, and includes such dosage forms as gel, cream, ointment, emulsion, suspension, solution, drops, lotion, spray or foam. In the present invention the topical formulation is in the form of an ointment.

The term "ointment" is a smooth oily substance which is rubbed on the skin for medicinal purposes or as a cosmetic. Ointments consist of single-phase bases in which drugs are dispersed. Ointment bases can consist of liquid paraffins or vegetable oils with emulsifying agents (water-emulsifying ointments) or without emulsifying agents (hydrophobic ointments), or with water-soluble bases, such as macrogols (hydrophilic ointments).

The term "therapeutically effective amount" refers to an amount, which achieves a desired effect, when administered to a living subject.

The term "active ingredient" or "active pharmaceutical ingredient" means any component that is intended to furnish pharmacological activity or other direct effect in the diagnosis, cure, mitigation, treatment, or prevention of disease, or to affect the structure or any function of the body of man or other animals. In the present invention, the active ingredient is tacrolimus or its pharmaceutically acceptable salts, esters, and solvates thereof.

Tacrolimus may be a free or pharmaceutically acceptable salt form, or a solvate such as hydrate or analog thereof. Since a salt form, a solvate or an analog of tacrolimus, especially tacrolimus monohydrate has a similar pharmacological activity to tacrolimus in a free form, the term "tacrolimus", as used in the present application and claims, means any or all of them. The term "tacrolimus" also includes tacrolimus in crystalline phase, noncrystalline phase or semicrystalline phase.

In this invention, weight percentages (w/w) refer to the weight of the component relative to weight of the composition.

Since tacrolimus dissolves poorly in water and lipid solvent, a preparation comprising it requires solubilizers capable of solubilizing tacrolimus.

Accordingly, several attempts has been made to prepare solid solution, preferably in the form of solid dispersions as also disclosed early in the development of tacrolimus formulations by Hone et al, Transplantation Proceedings, Vol XIX, No 5, Suppl 6 (October), 1987: pp 17-22, which discloses solid dispersions with different formulations. "Establishment of new preparation method for solid dispersion formulation of tacrolimus" by YAMASHITA Kazunari et al, International Journal of Pharmaceutics 2003, vol. 267, no1-2, pp. 79-91 discloses an improved solvent method in order to prevent the use of dichloromethane.

In the prior art, it is known that the active ingredient tacrolimus decomposes during long-term storage. The presence of degradation products in a pharmaceutical formulation, including a pharmaceutical composition comprising tacrolimus as an active pharmaceutical ingredient, is highly undesirable, since it imposes an increased risk to the patients. Increased impurity level might produce some unknown adverse biological effects thus compromising the safety of the product and become unfit for human consumption.

Further, very strict regulatory restrictions exists regarding impurities present in a pharmaceutical formulation, both in a newly prepared pharmaceutical formulation, and in pharmaceutical formulations upon storage, i.e. during their shelf-life. Accordingly, it is necessary to monitor and document the formation of any possible degradation product stemming from the pharmaceutical formulation itself, notably any degradation product arising from the active ingredient, and to either control the amount of degradation product present in the formulation during shelf-life, or to prevent or reduce the formation of degradation product during manufacturing or the shelf-life of the formulation, depending on the nature of each degradation product that may be present or in the pharmaceutical formulation product.

The degradation product limit set forth in the International Conference of Harmonization (ICH) guidelines (ICH Topic 3 Q B (R2): Note for Guidance on Impurities in New Drug Products, CPMP/ICH/2738/99, June 2006; www.ema.eu.int) depends on the amount of drug to be administered daily. For a maximum daily dose ranging from 10 to 100 mg, the ICH limit is 0.5% for any other unspecified degradation product.

The ICH guideline also indicates that stress testing is designed to help "determine the intrinsic stability of the molecule by establishing degradation pathways in order to identify the likely degradation products and to validate the stability indicating power of the analytical procedures used". Elevated thermal conditions are often used to accelerate chemical degradation processes that will occur at room temperature upon storage. In the present invention; thermal, 80°C, stress condition test was used to predict stability problems.

The topical preparation of the present invention may comprise commonly used additives, such as emulsifying agent, wetting agent, dispersant, plasticizer, pH regulator, absorbent, gelator, preservative, filler, preserving agent, antiseptic, pigment, flavoring agent, freshener, thickener, antioxidant, skin-lightening agent, ultraviolet absorber.

Propylene carbonate is used mainly as a polar solvent in oral and topical pharmaceutical formulations. The propylene carbonate acts as a carrier and permeation enhancer for the drugs. The absence of water limits the chances of chemical degradation due to hydrolysis or pH incompatibility.

Paraffin is mainly used in topical pharmaceutical formulations as a component of cream and ointments. In ointments, it may be used to increase the melting point of a formulation or to add stiffness.

The present inventors found that the weight ratio of tacrolimus or its salts to solvent, especially propylene carbonate, significantly affects the stability of the resulting formulation. Especially, the present inventors found that, if tacrolimus or its salts to propylene carbonate were formulated with using a specific weight ratio thereof, improved stability accomplished, thereby being able to obtain an ointment more stable even at elevated temperature stress condition.

Therefore, it is an object of the present invention to provide a pharmaceutical composition for topical administration comprising tacrolimus to propylene carbonate in a specific weight ratio.

The following examples are for the purpose of illustration of the invention only and are not intended in any way to limit the scope of the present invention.

### Example 1. Formulation and preparation method of Tacrolimus 0.1% Ointment

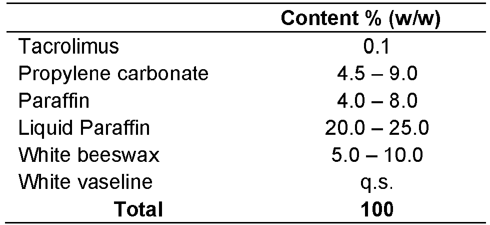

The oil phase components of paraffin, liquid paraffin, white beeswax and white vaseline are heated up to the entire melt in a mechanical stirrer at 70°C. On the other side, propylene carbonate is heated up to 70°C, tacrolimus is added into the hot propylene carbonate and they are mixed until dissolved. When the both mixtures are at 70°C, tacrolimus mixture is added into oil phase. Continue stirring until the product temperature reaches 25°C. The final product is homogenized and packed.

### Example 2. Compositions tested for determining the ratios of tacrolimus to propylene carbonate when the concentration of tacrolimus is 0.1 % (w/w) based on the total composition

**Table 1. Comparative degradation products for Test products (Test 0.1-1 to Test 0.1-5) initially and after 2 days at 80°C stres condition**

| **0.1 %** | **Ratio of Tacrolimus : Propylene carbonate (w/w)** | **Propylene carbonate % (w/w)** | **Any Other Unspecified Degradation Product (%)** | |
|---|---|---|---|---|
| | | | **Initially** | **Stres Condition (80°C for 2 days)** |
| **Test 0.1-1** | 1 : 90 | 9.0 | ND | 1.8 % |
| **Test 0.1-2** | 1 : 75 | 7.5 | ND | 1.9 % |
| **Test 0.1-3** | 1 : 60 | 6.0 | ND | 2.1 % |
| **Test 0.1-4** | 1 : 55 | 5.5 | ND | 3.4 % |
| **Test 0.1-5** | 1 : 50 | 5.0 | ND | 6.3 % |

| | | | | |
|---|---|---|---|---|
| ** ND: Not deteceted.* | | | | |

The test products were prepared in the same procedures as in Example 1, by fixing the concentration of tacrolimus to 0.1 %(w/w) and adjusting the amount of propylene carbonate as shown by the ratios in above table.

The initial amount of degradation products in each formulation was measured; and then the amount thereof was also measured after storing at 80°C for 2 days. The results are shown in table above.

As shown in table above, when the concentration of tacrolimus is 0.1 % (w/w) based on the total composition, chemical stability is obtained at the time a weight ratio of tacrolimus to propylene carbonate is 1:60 to 1:90 (w/w) based on the total composition. Therefore, the propylene carbonate weight percent is 6.0% to 9.0% (w/w) based on the total composition.

On the other hand, when the weight ratio of tacrolimus to propylene carbonate is higher than 1:90 (w/w), viscosity of the drug product would be low and therefore it is not applicable for ointment formulation.

### Example 3. Preparation method of Tacrolimus 0.03% Ointment

| | **Content % (w/w)** |
|---|---|
| Tacrolimus | 0.03 |
| Propylene carbonate | 4.5 - 9.0 |
| Paraffin | 4.0 - 8.0 |
| Liquid Paraffin | 20.0 - 25.0 |
| White beeswax | 5.0 - 10.0 |
| White vaseline | q.s. |
| **Total** | **100** |

The oil phase components of paraffin, liquid paraffin, white beeswax and white Vaseline are heated up to the entire melt in a mechanical stirrer at 70°C. On the other side, propylene carbonate is heated up to 70°C, tacrolimus is added into the hot propylene carbonate and they are mixed until dissolved. When the both mixtures are at 70°C, tacrolimus mixture is added into oil phase. Continue stirring until the product temperature reaches 25°C. The final product is homogenized and packed.

### Example 4. Compositions tested for determining the ratios of tacrolimus and propylene carbonate when the concentration of tacrolimus is 0.03% (w/w) based on the total composition

**Table 2. Comparative degradation products for Test products (Test 0.03-1 to Test 0.03-6) initially and after 2 days at 80°C stres condition**

| **0.03 %** | **Ratio of Tacrolimus** : **Propylene carbonate (w/w)** | **Propylene carbonate % (w/w)** | **Any Other Degradation Product (%)** | |
|---|---|---|---|---|
| | | | **Initially** | **Stres Condition (80°C for 2 days)** |
| **Test 0.03-1** | 1 : 300 | 9.0 | DE | 2.3 % |
| **Test 0.03-2** | 1 : 250 | 7.5 | DE | 2.3 % |
| **Test 0.03-3** | 1 : 200 | 6.0 | DE | 2.5 % |
| **Test 0.03-4** | 1 : 183 | 5.5 | DE | 3.9 % |
| **Test 0.03-5** | 1 : 166 | 5.0 | DE | 7.2 % |

The test products were prepared in the same procedures as in Example 3, by fixing the concentration of tacrolimus to 0.03 % (w/w) and adjusting the amount of propylene carbonate as shown by the ratios in above table.

The initial amount of degradation products in each formulation was measured; and then the amount thereof was also measured after storing at 80°C for 2 days. The results are shown in table above.

As shown in table above, when the concentration of tacrolimus is 0.03 % (w/w) based on the total composition, chemical stability is obtained at the time a weight ratio of tacrolimus to propylene carbonate is 1:200 to 1:300 (w/w). Therefore, the propylene carbonate weight percent is 6.0% to 9.0% (w/w) based on the total composition.

On the other hand, when the weight ratio of tacrolimus to propylene carbonate is higher than 1:300 (w/w), viscosity of the drug product would be low and therefore it is not applicable for this ointment formulation.

### Stability Studies

In order to examine the physical and chemical stability of the products, stability test was assessed under accelerated condition (40°C±2°C, 75% Relative humidity, RH±5%) and ambient temperature condition (25°C±2°C/ 60±5% Relative Humidity, RH±5%) for Test products (Test 0.1-1 to Test 0.1-3 and Test 0.03-1 to Test 0.03-3).

Test products are physically stable after 6 months under accelerated condition and after 12 months under ambient temperature condition. Physical stability was tested as the composition appears as a homogeneous ointment with no gross apparent rheological or appearance changes from initially (t=0). Preferably, the composition is physically stable for 6 months at 40°C±2°C, 75% Relative humidity (RH±5%) and 12 moths at 25°C±2°C/ 60±5% Relative Humidity (RH±5%).

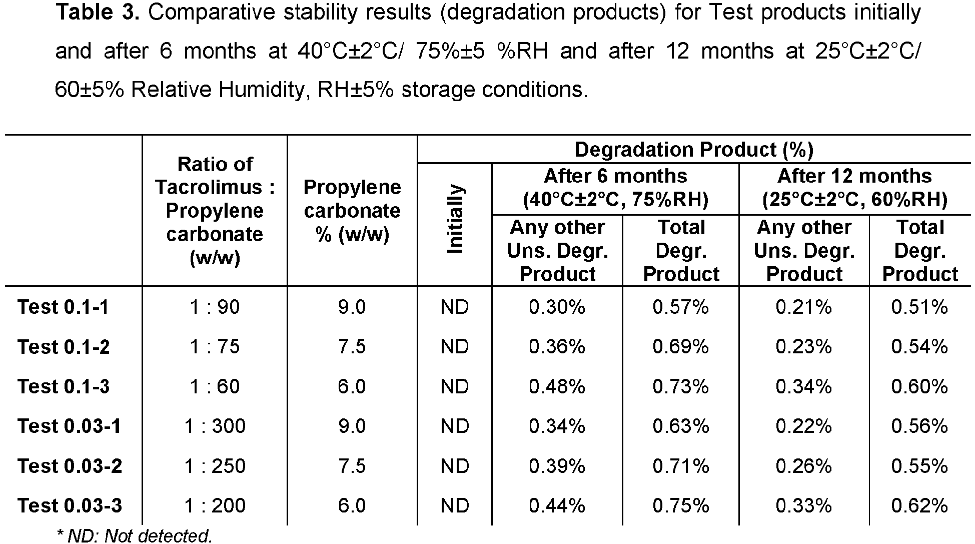

The stability results above (Table 3) show that, test products are chemically stable after 6 months under accelerated condition and after 12 months under ambient temperature condition. As indicated at EP2345414B1; for stability test, test product prepared with triacetin and reference product was stored at 3°C and 30°C for one week. The results given in this patent are any other unspecified degradation product 2.58% for Test product and 2.88% for Reference product after 1 week storage at 30°C. In this manner, the present invention is much more stable than test product prepared with triacetin and reference product (Protopic Ointment).

Surprisingly, it has been found that formation of degradation product is decreased with the present invention by using specific ratio of propylene carbonate in the formulation. Considering both the degradation and the viscosity of the product in the studies, the composition containing propylene carbonate in the range of 6.0%-7.0% is the main composition of the invention.

According to main embodiment of the invention, storage stable topical ointment compositions for treating atopic dermatisis are provided by the present invention comprising therapeutically effective amount of 0.1% - 0.03% tacrolimus or its pharmaceutically acceptable salts, esters, and solvates thereof as active ingredient and propylene carbonate as a solvent, wherein the weight ratio of propylene carbonate is in the range of 6.0% to 7.0% (w/w) based on the total composition.

## Claims

1. A pharmaceutical ointment composition, comprising 0.1% or 0.03% (w/w) tacrolimus or its pharmaceutically acceptable salt thereof, wherein the amount of propylene carbonate is 6.0% to 7.0% (w/w) based on the total composition.

2. The pharmaceutical composition according to claim 1, wherein the composition comprises tacrolimus, propylene carbonate, paraffin, white beeswax and white vaseline.

3. The pharmaceutical composition according to claim 2, wherein the composition is essentially free of stabilizing agent.

4. The pharmaceutical composition according to any of the preceeding claims, wherein the ointment composition comprises less than 0.5 % (w/w) of any other unspecified degradation product after 6 months of storage at 40°C±2°C/ 75%±5 %Relative Humidity.

## Patentansprüche

1. Pharmazeutische Salbenzusammensetzung, umfassend 0,1 % oder 0,03 % (Gew./Gew.) Tacrolimus oder ein pharmazeutisch annehmbares Salz davon, wobei die Menge an Propylencarbonat 6,0 % bis 7,0 % (Gew./Gew.), bezogen auf die Gesamtzusammensetzung, beträgt.

2. Pharmazeutische Zusammensetzung nach Anspruch 1, wobei die Zusammensetzung Tacrolimus, Propylencarbonat, Paraffin, weißes Bienenwachs und weiße Vaseline umfasst.

3. Pharmazeutische Zusammensetzung nach Anspruch 2, wobei die Zusammensetzung im Wesentlichen frei von einem Stabilisierungsmittel ist.

4. Pharmazeutische Zusammensetzung nach einem der vorhergehenden Ansprüche, wobei die Salbenzusammensetzung weniger als 0,5 % (Gew./Gew.) eines anderen nicht spezifizierten Abbauprodukts nach 6-monatiger Lagerung bei 40 °C ± 2 °C/75 % ± 5 % relativer Luftfeuchtigkeit umfasst.

## Revendications

1. Composition pharmaceutique sous forme de pommade, comprenant 0,1 % ou 0,03 % (en poids) de tacrolimus ou d'un sel pharmaceutiquement acceptable de celui-ci, dans laquelle la quantité de carbonate de propylène est comprise entre 6,0 % et 7,0 % (en poids) par rapport à la composition totale.

2. Composition pharmaceutique selon la revendication 1, dans laquelle la composition comprend du tacrolimus, du carbonate de propylène, de la paraffine, de la cire d'abeille blanche et de la vaseline blanche.

3. Composition pharmaceutique selon la revendication 2, dans laquelle la composition est essentiellement exempte d'agent stabilisant.

4. Composition pharmaceutique selon l'une quelconque des revendications précédentes, dans laquelle la composition de pommade comprend moins de 0,5 % (p/p) de tout autre produit de dégradation non spécifié après 6 mois de stockage à 40 °C ± 2 °C/75 % ± 5 % d'humidité relative.
